# EUROPEAN PATENT SPECIFICATION

(11) **EP 2 029 065 B1**
(45) Date of publication and mention of the grant of the patent: **15.03.2017**
(21) Application number: 07783887.8
(22) Date of filing: 17.05.2007
(51) Int. Cl.: A61F 2/954

(54) **STENT AND CATHETER ASSEMBLY**
STENT UND KATHETERANORDNUNG
ENSEMBLE STENT ET CATHÉTER

(30) Priority: 07.06.2006 US 811699 P; 16.05.2007 US 749619
(43) Date of publication of application: 04.03.2009
(73) Proprietor: Abbott Cardiovascular Systems Inc., Santa Clara, CA 95054 (US)
(72) Inventor: WILLIAMS, Mark A., Redwood City, California 04065 (US)
(74) Representative: Boult Wade Tennant
(86) International application number: PCT/US2007/069161
(87) International publication number: WO 2007/143360

(56) References cited:
- WO-A-99/03426
- WO-A-03/105922
- WO-A-2004/093968
- WO-A1-00/74595
- WO-A2-02/091951
- US-A- 5 749 825
- US-A1- 2004 088 007

## Description

### BACKGROUND OF THE INVENTION

The invention relates to stents and stent delivery and deployment assemblies for use at a bifurcation and, more particularly, one or more stents for repairing bifurcations, blood vessels that are diseased, and an apparatus for delivery and implantation of the stents.

Stents conventionally repair blood vessels that are diseased. Stents are generally hollow and cylindrical in shape and have terminal ends that are generally perpendicular to their longitudinal axis. In use, the conventional stent is positioned at the diseased area of a vessel and, after deployment, the stent provides an unobstructed pathway for blood flow.

Repair of vessels that are diseased at a bifurcation is particularly challenging since the stent must be precisely positioned, provide adequate coverage of the disease, provide access to any diseased area located distal to the bifurcation, and maintain vessel patency in order to allow adequate blood flow to reach the myocardium. Therefore, the stent must provide adequate coverage to the diseased portion of the bifurcated vessel, without compromising blood flow, and extend to a point within and beyond the diseased portion. Where the stent provides coverage to the vessel at the diseased portion, yet extends into the vessel lumen at the bifurcation, the diseased area is repaired, but blood flow may be compromised in other portions of the bifurcation. Unapposed stent elements may promote lumen compromise during neointimal formation and healing, producing restenosis and requiring further procedures. Moreover, by extending into the vessel lumen at the bifurcation, the stent may block access to further interventional procedures.

Conventional stents are designed to repair areas of blood vessels that are removed from bifurcations, and, therefore are associated with a variety of problems when attempting to use them to treat lesions at a bifurcation. Conventional stents are normally deployed so that the entire stent is either in the parent vessel or the proximal portion of the stent is in the parent vessel and the distale portion is located in the side branch vessel. In both cases, either the side branch vessel (former case) or the parent vessel (latter case), would become "jailed" by the stent struts. This technique repairs one vessel at the bifurcation at the expense of jailing or obstructing the alternate vessel. Blood flow into the jailed vessel would be compromised as well as future access and treatment into the distal portion of the jailed vessel.

Alternatively, access into a jailed vessel can be attained by carefully placing a guide wire through the stent, and subsequently tracking a balloon catheter through the stent struts. The balloon could then be expanded, thereby deforming the stent struts and forming an opening into the previously jailed vessel. The cell to be spread apart must be randomly and blindly selected by re crossing the deployed stent with a guide wire. The drawback with this approach is that there is no way to determine or guarantee that the main vessel stent struts are properly oriented with respect to the side branch or that an appropriate stent cell has been selected by the wire for dilatation. The aperture created often does not provide a clear opening and creates a major distortion in the surrounding stent struts. A further drawback with this approach is that there is no way to tell if the main-vessel stent struts have been properly oriented and spread apart to provide a clear opening for stenting the side branch vessel. This technique also causes stent deformation to occur in the area adjacent to the carina, pulling the stent away from the vessel wall and partially obstructing flow in the originally non jailed vessel. Deforming the stent struts to regain access into the previously jailed strut is also a complicated and time consuming procedure associated with attendant risks to the patient and is typically performed only if considered an absolute necessity. Vessels which supply a considerable amount of blood supply to the myocardium and may be responsible for the onset of angina or a myocardial infarct would necessitate the subsequent strut deformation in order to reestablish blood flow into the vessel. The risks of procedural complications during this subsequent deformation are considerably higher than stenting in normal vessels. The inability to place a guide wire through the jailed lumen in a timely fashion could restrict blood supply and begin to precipitate symptoms of angina or even cardiac arrest. In addition, platelet agitation and subsequent thrombus formation at the jailed site could further compromise blood flow into the side branch.

Plaque shift is also a phenomena which is of concern when deploying a stent across a bifurcation. Plaque shift occurs when treatment of disease or plaque in one vessel causes the plaque to shift into another location. This is of greatest concern when the plaque is located on the carina or the apex of the bifurcation. During treatment of the disease the plaque may shift from one side of the carina to the other thereby shifting the obstruction from one vessel to the alternate vessel.

In another prior art method of implanting stents, a "T" stent procedure includes implanting a stent in the side branch ostium of the bifurcation followed by stenting the main vessel across the side branch and subsequently deforming the struts as previously described, to allow blood flow and access into the side branch vessel. Alternatively, a stent is deployed in the parent vessel and across the side branch origin followed by subsequent strut deformation as previously described, and finally a stent is placed into the side branch vessel. T stenting may be necessary in some situations in order to provide further treatment and additional stenting in the side branch vessel. This is typically necessitated when the disease is concentrated at the origin of the jailed vessel. This procedure is also associated with the same issues and risks previously described when stenting only one vessel and deforming the struts through the jailed vessel. In addition, since a conventional stent generally terminates at right angles to its longitudinal axis, the use of conventional stents to treat the origin of the previously jailed vessel (typically the side branch vessel) may result in blocking blood flow of the originally non jailed vessel (typically the parent vessel) or fail to provide adequate coverage of the disease in the previously jailed vessel (typically a side branch vessel). The conventional stent might be placed proximally in order to provide full coverage around the entire circumference of the side branch, however this leads to a portion of the stent extending into the pathway of blood flow of the parent vessel. The conventional stent might alternatively be placed distally to, but not entirely overlaying the circumference of the origin of the side branch to the diseased portion. Such a position of the conventional stent results in a bifurcation that does not provide full coverage or has a gap on the proximal side (the origin of the side branch) of the vessel and is thus not completely repaired. The only conceivable situation that the conventional stent, having right angled terminal ends, could be placed where the entire circumference of the ostium is repaired without compromising blood flow, is where the bifurcation is formed of right angles. In such scenarios, extremely precise positioning of the conventional stent is required. This extremely precise positioning of the conventional stent may result with the right angled terminal ends of the conventional stent overlying the entire circumference of the ostium to the diseased portion without extending into a side branch, thereby repairing the right angled bifurcation.

To circumvent or overcome the problems and limitations associated with conventional stents in the context of repairing diseased bifurcated vessels, a stent that consistently overlays most of the diseased area of the bifurcation and provides adequate access to distal disease without subjecting the patient to any undue risks may be employed. Such a stent would have the advantage of providing adequate coverage at the proximal edge of the origin of the side branch such that a conventional stent which terminates at right angles to its longitudinal axis can be deployed in the side branch or alternate vessel without leaving a significant gap at the origin of the side branch. In addition, such a stent would allow access to all portions of the bifurcated vessel should further interventional treatment be necessary.

In another prior art method for treating bifurcated vessels, commonly referred to as the "Culotte technique," the side branch vessel is first stented so that the stent protrudes into the main or parent vessel. A dilatation is then performed in the main or parent vessel to open and stretch the stent struts extending across the lumen from the side branch vessel. Thereafter, a stent is implanted in the side branch so that its proximal end overlaps with the parent vessel. One of the drawbacks of this approach is that the orientation of the stent elements protruding from the side branch vessel into the main vessel is completely random. In addition excessive metal coverage exists from overlapping strut elements in the parent vessel proximal to the carina area. Furthermore, the deployed stent must be recrossed with a wire blindly and arbitrarily selecting a particular stent cell. When dilating the main vessel the stent struts are randomly stretched, thereby leaving the possibility of restricted access, incomplete lumen dilatation, and major stent distortion.

In another prior art procedure, known as "kissing" stents, a stent is implanted in the main vessel with a side branch stent partially extending into the main vessel creating a double-barrelled lumen of the two stents in the main vessel distal to the bifurcation. Another prior art approach includes a so-called "trouser legs and seat" approach, which includes implanting three stents, one stent in the side branch vessel, a second stent in a distal portion of the main vessel, and a third stent, or a proximal stent, in the main vessel just proximal to the bifurcation.

Exemplary stent and catheter assemblies are disclosed in WO02091951 and in WO0074595

All of the foregoing stent deployment assemblies suffer from the same problems and limitations. Typically, there is uncovered intimal surface segments on the main vessel and side branch vessels between the stented segments or there is excessive coverage in the parent vessel proximal to the bifurcation. An uncovered flap or fold in the intima or plaque will invite a "snowplow" effect, representing a substantial risk for sub acute thrombosis, and the increased risk of the development of restenosis. Further, where portions of the stent are left unapposed within the lumen, the risk for subacute thrombosis or the development of restenosis again is increased. The prior art stents and delivery assemblies for treating bifurcations are difficult to use and deliver making successful placement nearly impossible. Further, even where placement has been successful, the side branch vessel can be "jailed" or covered so that there is impaired access to the stented area for subsequent intervention. The present invention solves these and other problems as will be shown.

The intravascular stent has a pattern or configuration that permits the stent to be tightly compressed or crimped onto a catheter to provide an extremely low profile and to prevent relative movement between the stent and the catheter. The stent also is highly flexible along its longitudinal axis to facilitate delivery through tortuous body lumens, but is stiff and stable enough radially in its expanded condition to maintain the patency of a body lumen such as an artery when the stent is implanted therein.

The stent generally includes a plurality of cylindrical rings that are interconnected to form the stent.

Each of the cylindrical rings making up the stent have a proximal end and a distal end and a cylindrical plane defined by a cylindrical outer wall surface that extends circumferentially between the proximal end and the distal end of the cylindrical ring. Generally the cylindrical rings have a serpentine or undulating shape which includes at least one U-shaped element, and typically each ring has more than one U shaped element. The cylindrical rings are interconnected by at least one undulating link which attaches one cylindrical ring to an adjacent cylindrical ring. The undulating links are highly flexible and allow the stent to be highly flexible along its longitudinal axis. At least some of the undulating links have a curved portion that extends transverse to the stent longitudinal axis for a predetermined distance that coincides with one of the U-shaped elements. More specifically, the curved portion extends in a transverse manner such that it would intersect with the corresponding U-shaped element, however, the corresponding U-shaped element is shorter in length than other U-shaped elements in the same ring. Thus, when the stent is compressed or crimped onto the catheter, the curved portions do not overlap or intersect with the adjacent U-shaped element since that element is shorter in length than similar U-shaped elements in the particular ring. In this manner, the stent can be compressed or crimped to a much tighter or smaller diameter onto the catheter which permits low profile delivery as well as a tight gripping force on the catheter to reduce the likelihood of movement between the stent and the catheter during delivery and prior to implanting the stent in the vessel.

The undulating links may take various configurations but in general have an undulating or serpentine shape. The undulating links can include bends connected by substantially straight portions wherein the substantially straight portions are substantially perpendicular to the stent longitudinal axis.

Not only do the undulating links that interconnect the cylindrical rings provide flexibility to the stent, but the positioning of the links also enhances the flexibility by allowing uniform flexibility when the stent is bent in any direction along its longitudinal axis. Uniform flexibility along the stent derives in part from the links of one ring being circumferentially offset from the links in an adjacent ring. Further, the cylindrical rings are configured to provide flexibility to the stent in that portions of the rings can flex or bend and tip outwardly as the stent is delivered through a tortuous vessel.

The cylindrical rings typically are formed of a plurality of peaks and valleys, where the valleys of one cylindrical ring are circumferentially offset from the valleys of an adjacent cylindrical ring. In this configuration, at least one undulating link attaches each cylindrical ring to an adjacent cylindrical ring so that at least a portion of the undulating links is positioned within one of the valleys and it attaches the valley to an adjacent peak.

While the cylindrical rings and undulating links generally are not separate structures, they have been conveniently referred to as rings and links for ease of identification. Further, the cylindrical rings can be thought of as comprising a series of U's, W's and Y-shaped structures in a repeating pattern. Again, while the cylindrical rings are not divided up or segmented into U's, W's and Y's, the pattern of the cylindrical rings resemble such configuration. The U's, W's and Y's promote flexibility in the stent primarily by flexing and by tipping radially outwardly as the stent is delivered through a tortuous vessel.

The undulating links are positioned so that the curved portion of the link is outside the curved part of the W-shaped portion. Since the curved portion does not substantially expand (if at all) when the stent is expanded, it will continue to provide good vessel wall coverage even as the curved part of the W-shaped portion spreads apart as the stent is expanded. The curved portion of the link extends in a direction transverse to the stent longitudinal axis for a distance that positions it adjacent and proximal to the peak of a U-shaped element. These U-shaped elements have struts that are shorter than the struts of the other U shaped elements in the same cylindrical ring so that as the stent is compressed the curved portion of the link does not overlap the adjacent U-shaped element.

The number and location of undulating links that interconnect adjacent cylindrical rings can be varied as the application requires. Since the undulating links typically do not expand when the cylindrical rings of the stent expand radially outwardly, the links are free to continue to provide flexibility and to also provide a scaffolding function to assist in holding open the artery. Importantly, the addition or removal of the undulating links has very little impact on the overall longitudinal flexibility of the stent. Each undulating link is configured so that it promotes flexibility whereas some prior art connectors actually reduce flexibility of the stent.

The cylindrical rings of the stent are plastically deformed when expanded when the stent is made from a metal that is balloon expandable. Typically, the balloon-expandable stent is made from a stainless steel alloy, cobalt chromium, tungsten, polymers, or similar material.

Similarly, the cylindrical rings of the stent expand radially outwardly when the stent is formed from superelastic alloys, such as nickel-titanium (NiTi) alloys. In the case of superelastic alloys, the stent expands upon application of a temperature change or when a stress is relieved, as in the case of a pseudoelastic phase change.

Because of the undulating configuration of the links, the stent has a high degree of flexibility along the stent axis, which reduces the tendency of stent fishscaling. Stent fishscaling can occur when the stent is bent and portions of the stent project outward when the stent is in the unexpanded condition. The undulating links reduce the likelihood of fishscaling.

Further, because of the positioning of the links, and the fact that the links do not expand or stretch when the stent is radially expanded, the overall length of the stent is substantially the same in the unexpanded and expanded configurations. In other words, the stent will not substantially shorten upon expansion.

The stent may be formed from a tube by laser cutting the pattern of cylindrical rings and undulating links in the tube. The stent also may be formed by laser cutting a flat metal sheet in the pattern of the cylindrical rings and links, and then rolling the pattern into the shape of the tubular stent and providing a longitudinal weld to form the stent.

The stent includes struts that make up the rings and links, the struts having either uniform cross-sections, or cross-sections having various widths and thicknesses.

### The Stent Delivery Catheter

The stent delivery catheter assembly for repairing bifurcated vessels includes an elongated catheter body which has a proximal catheter shaft, an intermediate section or mid-section, and a distal section. The catheter assembly contains an over-the-wire (OTW) guide wire lumen extending from the proximal catheter hub to one of the two distal tips of the distal end of the catheter. The catheter assembly also includes a rapid exchange (Rx) guide wire lumen which extends from the proximal end of the mid-section to the other of the two distal tips of the distal end of the catheter. The proximal catheter shaft also contains an inflation lumen which extends from the proximal hub of the proximal catheter shaft to the mid-section of the catheter and is in fluid communication with the inflation lumen contained within the mid-section. The mid-section contains lumens for both an OTW and an Rx guide wire lumen. The Rx guide wire lumen begins at about the proximal section of the intermediate shaft and extends to one of the distal tips of the distal catheter shaft. The OTW guide wire lumen extends through the intermediate section of the catheter and extends proximally to the catheter hub connected to the proximal catheter shaft and extends distally to one of the tips of the distal section of the catheter. The distal section of the catheter consists of two shafts extending from the distal end of the mid-shaft to the distal end of the catheter tips. One shaft has a balloon connected adjacent the distal end and a tip connected to the distal end of the balloon while the other shaft is devoid of a balloon. Each shaft contains a guide wire lumen extending therethrough. The shaft with the balloon includes an inflation lumen which is in fluid communication with the inflation lumen of the mid-shaft. One of the shafts of the distal section contains an Rx guide wire lumen, which extends proximally through the mid-section of the catheter and exits at about the proximal end of the mid-section of the catheter, the Rx guide wire lumen also extends distally to one of the tips of the distal section of the catheter. The second shaft of the distal section contains an OTW guide wire lumen, which extends proximally through the mid-section and proximal section of the catheter and exits at the proximal hub connected to the distal end of the proximal catheter section, the OTW guide wire lumen also extends distally to one of the tips of the distal section of the catheter. The balloon is connected to the catheter shaft such that the inflation lumen of the shaft is in fluid communication with the balloon and the guide wire lumen contained within the shaft extends through the center of the balloon. The proximal section of the balloon is sealed to the distal end of the shaft and the distal end of the balloon is sealed around the outside of the guide wire lumen or inner member running through the center of the balloon. The proximal and distal seals of the balloon allow for fluid pressurization and balloon inflation from the proximal hub of the catheter. The tip at the distal end of the shaft with the balloon extends further distally than the tip of balloon-less shaft and includes a coupler for receiving a guidewire extending through the balloon-less shaft so as to allow the two shafts to effectively be joined during delivery of the previously described stent.

The stent is crimped or compressed onto the balloon and a section of the balloon-less shaft such that the balloon extends through the distal opening and the proximal opening in the stent, while the balloon-less shaft extends through the proximal opening and the central opening of the stent. The resulting assembly has a profile that is much smaller than is possible when relying on a catheter with two balloons or a catheter with a bifurcated balloon to deliver a bifurcated stent.

There is also described an improvement on previously described polymeric radiopaque markers by enabling a polymer to be filled or doped with a considerably greater quantity of a radiopaque agent than has heretofore been achievable. The increased fill ratio nonetheless allows uniform pellets to be compounded and an extrusion with the appropriate wall thickness to be formed. The resulting marker provides an unprecedented combination of radiopacity and flexibility. Such marker would allow any of various intraluminal devices to be radiopaquely marked including, but not limited to, coronary and peripheral balloon catheters, stent delivery catheters, and guiding catheters as well as guidewires.

The marker relies on the use of radiopaque materials with a preselected particle shape and a preselected particle size distribution as well as the inclusion of one or more additives in the polymer/radiopaque agent blend. A multifunctional polymeric additive is added to the composition in order to enhance the wetting, adhesive and flow properties of the individual radiopaque particles by the polymer so as to cause each particle to be encapsulated by the polymer and thereby allow the polymer to form a continuous binder. An antioxidant may optionally be added in order to preserve the high molecular weight of the polymer matrix as it is exposed to the high temperatures and shear stresses associated with the compounding and extrusion processes.

While previous attempts to increase fill ratios have involved tungsten powder of relatively fine particle size, the present marker relies on the use of particles of increased size in order to achieve such end. An increase in particle size has been found to allow the polymer to more effectively function as a continuous binder and thereby increase ductility at a given fill ratio or maintain ductility at increased fill ratios. It has been found that in constraining the average particle size to at least 2 microns and limiting maximum particle size to about 20 microns provides the desired results. In the case of tungsten in Pebax, a fill ratio of about 91.3 weight percent (equivalent to 36.4 volume percent) or more is readily attainable for a polymeric marker formed in this way. In one arrangement, the fill ratio is about 90.8 weight percent (34.9 volume percent) to about 93.2 weight percent (42.7 volume percent). Additionally, it has been found that the process by which the tungsten powder is produced has a considerable effect on both particle size distribution as well as the shape of the individual particles. Tungsten powder produced by either a "pusher" process or "atomization" process, then milled and classified has been found to provide discrete particles having a more equiaxed shape and size respectively and are therefore more ideally suited for the present purposes than powders produced employing a "rotary" process.

The marker is manufactured by first tumble mixing the polymer resin with a pelletized wetting agent, such as maleic anhydride graft polyolefin resin (MA-g-PO), and an antioxidant and then introducing the mixture into the primary feeder of a twin screw extruder. The mixture is fed in at a controlled mass flow rate and conveyed down the barrel length as it is heated above its melting temperature and blended. At a point downstream, tungsten powder is introduced into the mix at a controlled mass flow rate via a secondary feeder. The tungsten powder and the molten ingredients become intimately intermixed as they are conveyed downstream and discharged through a die as molten strands which are cooled in water and subsequently pelletized. The markers are subsequently formed by extruding the tungsten filled polymer onto a continuous beading of PTFE, and drawn down to yield the desired wall thickness. The extrusion tolerances are such that the outer diameter of the extrusion can vary by as much as 0.025mm (0.001 inch), which is large enough to significantly affect the radiopacity and profile of the finished marker. Consequently, in a preferred arrangement, the extrusion is hot die necked (i.e., the extruded tube on a support mandrel, such as the PTFE beading, is pulled through a heated die to take on the die size), to resize the outer diameter and provide the desired wall thickness. The extrusion is then cut to the desired lengths, preferably with the beading still in place so as to provide support. Removal of the beading remnant then allows the marker to be slipped onto the medical device or component thereof to be marked and melt bonded in place. The polymeric marker is typically a closed, solid-walled band with a tubular or annular shape. However, the polymeric marker can have a variety of suitable shapes. Reliance on melt bonding obviates the need for the marker to completely surround the underlying device. Markers can for example be longitudinally split in half to form two markers of C-shaped cross-section. Or, solid strands of extruded marker material may be melt bonded to one side to form one or more longitudinal stripes or helical patterns.

During melt bonding, the polymeric marker is heated to an elevated temperature sufficient to melt the polymeric material to produce the melt bond. However, in one preferred arrangement, the elevated temperature is low enough to minimize or prevent flowing of the polymeric material of the marker. Consequently, the double wall thickness (inner diameter minus outer diameter) of the polymeric marker after melt bonding is equal to or not substantially less than (i.e., not more than about 5 to about 25% less than) the double wall thickness prior to melt bonding. Although some rounding of the edges of the polymeric marker may occur during melt bonding, the maximum wall thickness of the polymeric marker is preferably not reduced during the melt bonding. The marker radiopacity is strongly affected by both the percent loading of radiopaque material and the final wall thickness of the marker. Consequently, the percent loading of radiopaque material and the wall thickness of the finished marker are carefully controlled to provide the desired radiopacity. If the wall thickness of the polymeric marker is less than the required minimum wall thickness, the radiopacity of the marker will be too low. On the other hand, if the outer diameter/wall thickness of the polymeric marker is greater than the required maximum outer diameter/wall thickness, the large profile and added stiffness of the marker will disadvantageously affect catheter performance. Thus, the hot die necked extrusion has an outer diameter and wall thickness which is within the minimum and maximum desired values, so that little or no thinning of the marker is required during melt bonding of the marker to provide the desired outer diameter. As a result, a decrease in wall thickness which can reduce the radiopacity of the finished marker is prevented or minimized during bonding. In one arrangement the polymeric marker is adhesively bonded to the medical device or component, and as a result, the wall thickness of the marker is not affected by the bonding process.

Due to its high radiopacity, flexibility and melt bondability, the marker is readily attached to for example the distal tip of a balloon catheter or a guidewire. The attachment of radiopaque marker at multiple-locations on one or more catheter balloons on a bifurcated catheter delivery system with known separation distances impart a measurement capability to the catheter that allows a physician to quickly and easily determine side branch vessel location and access. Markers are positioned so as to enable a physician to determine both the position and orientation of the device. Accordingly, the balloon has a total of three markers attached thereto, corresponding with features of the stent crimped thereto, namely the distal and proximal ends of the stent and the side branch portal. The balloon-less shaft has two markers attached thereto, one at its distal end and another just distal to the side branch portal of the stent crimped thereto.

### Delivering And Implanting The Stent

The method of delivering and implanting the stent mounted on the catheter assembly is also described. The bifurcated catheter assembly of the present invention provides two separate shafts in parallel which are advanced into separate passageways of an arterial bifurcation at which point the balloon is inflated to expand and implant the stent More specifically, the catheter assembly is advanced through a guiding catheter (not shown) until the distal end of the catheter assembly reaches the ostium to the coronary arteries. An Rx guide wire is advanced out of the Rx shaft and into the coronary arteries to a point distal of the bifurcation or target site. In a typical procedure, the Rx guide wire will already be positioned in the main vessel after a pre dilatation procedure. The catheter assembly is advanced over the Rx guide wire so that the catheter distal end is just proximal to the opening to the side branch vessel. Up to this point in time, the OTW guide wire (or mandrel or joining wire) remains within the catheter assembly and within the coupler so that the shaft with the balloon and the balloon-less shaft remain adjacent to one another to provide a low profile. As the catheter assembly is advanced to the bifurcated area, the coupler moves axially relative to the distal end of the OTW guide wire (or mandrel or joining wire) a small distance (approximately 0.5 mm up to about 5.0 mm), but is not pulled completely out of the coupler, making it easier for the distal end of the catheter to negotiate tortuous turns in the coronary arteries. Thus, the slight axial movement of the coupler relative to the OTW guide wire (or mandrel or joining wire) distal end allows the tips to act or move independently, thereby increasing flexibility over the tips joined rigidly and aids in the smooth tracking of the catheter assembly over the Rx guide wire. The proximal end of the OTW guide wire is releasably attached to the proximal hub as previously described. The OTW guide wire (or mandrel or joining wire) is removed or withdrawn proximally from the coupler, thereby uncoupling the long balloon and the balloonless shaft. Thereafter, the OTW guide wire is advanced distally into the side branch vessel so that the catheter assembly can next be advanced distally over the Rx guide wire in the main vessel and the OTW guide wire in the side branch vessel. The separation between the Rx guide wire and the OTW guide wire allows the shaft with the balloon and the balloon-less shaft to separate slightly as the catheter assembly is further advanced over the Rx guide wire and the OTW guide wire. The catheter assembly advances distally until it reaches a point where the central opening on the stent is approximately adjacent to the opening to the side branch vessel, so that the catheter assembly can no longer be advanced distally since the stent is now pushing up against the opening to the side branch vessel. The three high percent tungsten/radiopaque markers that are disposed on the balloon and the two that are disposed on the balloon-less shaft aid in the proper positioning of the stent with respect to the bifurcation or target site by enabling the physician to readily discern the longitudinal as well as rotational orientation of the catheter and stent. Once the balloon and balloon-less shafts with the stent mounted thereon are in position in the main vessel and side branch vessel, the balloon is inflated to expand the stent in the main vessel whereby the presence of the balloon-less shaft between the expanded balloon and the stent in combination with the angling of the shaft by virtue of the bifurcation causes the side branch portal to be opened sufficiently to provide access for the subsequent balloon expansion procedure

As the catheter assembly is advanced through tortuous coronary arteries, over the Rx guide wire, the central opening of the stent may or may not always be perfectly aligned with the opening to the side branch vessel. If the central opening of the stent is in alignment with the opening to the side branch vessel it is said to be "in phase" and represents the ideal position for stenting the main branch vessel and the opening to the side branch vessel. When the central opening of the stent and the opening to the side branch vessel are not aligned it is said to be "out of phase" and depending upon how many degrees out of phase, the stent may require repositioning or reorienting so that the central opening more closely coincides with the opening to the side branch vessel. The orientation of the central opening of the stent with respect to the opening to the side branch vessel can range anywhere from in phase to a few degrees out of phase to 180° out of phase. If the central opening of the stent is more than 90° out of phase with respect to the opening to the side branch vessel, it may be difficult to position the radiopaque marker, and thus the linear or longitudinal position of the stent. When the central opening is in the out of phase position, the stent still can be implanted and the central opening will expand into the opening of the side branch vessel and provide adequate coverage. In cases where the system is more than 90° out of phase, the Rx and OTW guide wires will be crossed causing a distal torque to be applied to help the system to rotate in phase. In the event rotation does not occur, the system can be safely deployed with adequate coverage and support as long as the radiopaque markers located on the distal end of the catheter reach the proper positioning as can be detected under fluoroscopy. The unique and novel design of the catheter assembly and the stent of the present invention minimizes the misalignment so that the central opening of the stent generally can be aligned with the opening to the side branch vessel, and is capable of stenting the opening to the side branch vessel even if the central opening is out of phase from the opening of the side branch vessel.

After the stent has been expanded at the bifurcation, kissing balloons are introduced into the main vessel and through the partially opened side portal into the side branch where upon they are inflated to expand the side portal to thereby provide a clear blood flow path through both the main branch as well as the side branch.

After the stent has been implanted at the bifurcaton, if necessary a second stent can be implanted in the side branch vessel so that the second stent abuts the central opening of the stent of the present invention.

### BRIEF DESCRIPTION OF THE DRAWINGS

FIGURE 1 is a perspective view of the stent is an unexpanded configuration;
FIG. 2 is a flattened elevational view of the stent in FIG. 1;
FIG. 3 is an elevational view of the catheter assembly for delivering and implanting the stent ;
FIG. 4 is a cross-sectional view taken along lines 4-4 of FIG. 3;
FIG. 5 is a cross-sectional view taken along lines 5-5 of FIG. 3;
FIG. 6 is a cross-sectional view taken along lines 6-6 of FIG. 3;
FIG. 7 is a cross-sectional view taken along lines 7-7 of FIG. 3;
FIG. 8 is a longitudinal cross-sectional view of the coupler;
FIG. 9 is a longitudinal cross-sectional view depicting a portion of the catheter distal end including the radiopaque markers;
FIG. 10 is a schematic view of the catheter under radioscopy in an out-of-phase orientation;
FIG. 11 is a schematic view of the catheter under radioscopy in an in-phase orientation;
FIG. 12 is a schematic view of the catheter under radioscopy properly deployed;
FIG. 13 is an elevational view of the catheter assembly being advanced into the main vessel;
FIG. 14 is an elevational view of the catheter assembly in the main vessel prior to advancement into the side branch vessel;
FIG. 15 is an elevational view of the catheter assembly as the over the wire guide wire is being advanced into the side branch vessel;
FIG. 16 is an elevational view of the catheter assembly positioned in the main vessel and the over-the-wire guide wire advanced and positioned in the side branch vessel;
FIG. 17 is an elevational view of the catheter assembly advanced so that the long balloon is in the main vessel and a portion of the balloonless shaft is positioned in the side branch vessel;
FIG. 18 is an elevational view of a bifurcation depicting the stent implanted in the main vessel with side branch portal opened sufficiently for subsequent expansion;
FIG. 19 is an elevational view of a bifurcation depicting the stent implanted in the main vessel with side branch portal fully expanded; and
FIG. 20 is an elevational view of a bifurcation in which the stent is implanted in the main vessel, and a second stent is implanted in the side branch vessel.

### DETAILED DESCRIPTION OF THE PREFERRED EMBODIMENTS

The present invention includes a stent and stent delivery catheter assembly for treating bifurcations in, for example, the coronary arteries, veins, peripheral vessels and other body lumens.

The stent can be implanted in the main or side branch vessels to treat a number of disease configurations at a bifurcation, but not limited to, the hollowing:
1. Treatment of a parent or main vessel and the origin of the side branch at a bifurcation with any angle associated between the side branch and parent vessel.
2. Treatment of a parent vessel proximal to the carina and the side branch vessel simultaneously.
3. Treatment of the proximal vessel extending only into the origin of the side branch and the origin of the distal parent at the bifurcation.
4. Treatment of the area at the bifurcation only.
5. The origin of an angulated posterior descending artery.
6. The origin of an LV extension branch just at and beyond the crux, sparing the posterior descending artery.
7. The origin of a diagonal from the left anterior descending.
8. The left anterior descending at, just proximal to, or just distal to the diagonal origin.
9. The origin of a marginal branch of the circumflex.
10. The circumflex at, just proximal to, or just distal to the marginal origin.
11. The origin of the left anterior descending from the left main.
12. The origin of the circumflex from the left main.
13. The left main at or just proximal to its bifurcation.
14. Any of many of the above locations in conjunction with involvement of the bifurcation and an alternate vessel.
15. Any bifurcated vessels within the body where conventional stenting would be considered a therapeutic means of treatment proximal or distal to the bifurcation.

The present invention solves the problems associated with the prior art devices by providing a stent which adequately covers the main branch vessel and extends partially into the side branch vessel to cover the origin of the side branch vessel as well. There is also described a stent delivery catheter assembly and a method of crimping the stent on the catheter and delivering and implanting the stent in the body, especially the coronary arteries.

### The Stent

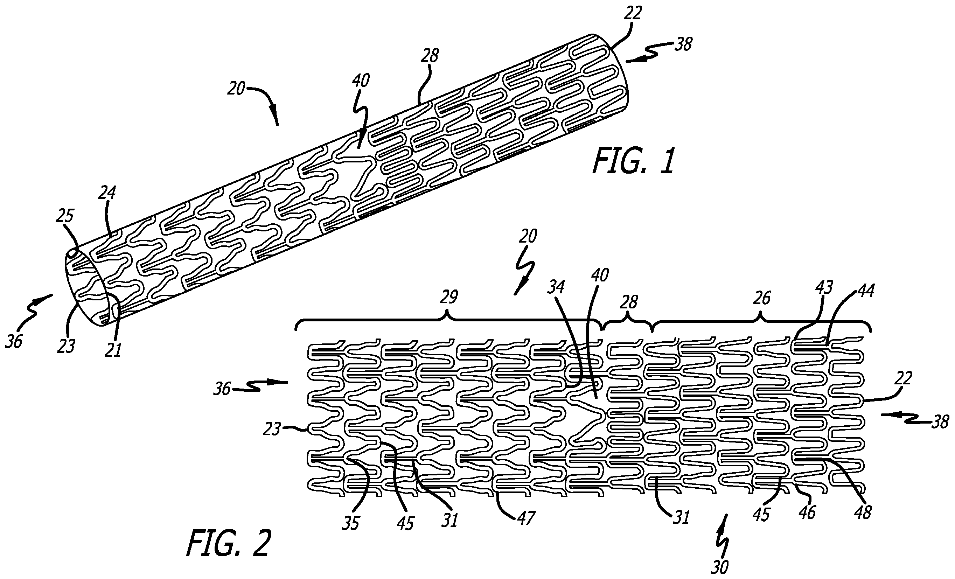

The stent pattern provides for vessel wall coverage of the main branch vessel and at least partial coverage of the origin of the side branch vessel. As is shown in Figs. 1 and 2, the stent 20 has a cylindrical body 21 that includes a proximal end 22 and a distal end 23. The stent has an outer surface 24 which contacts the vascular wall when implanted and an inner surface 25 through which blood flows when the stent is expanded and implanted. The stent can be described as having numerous connected rings 30 aligned along a common longitudinal axis of the stent. The rings are formed of undulating portions which include peaks 34 that are configured to be spread apart to permit the stent to be expanded to a larger diameter or compressed tightly toward each other to a smaller diameter when mounted on a catheter. The rings are connected to each other by at least one link 31 between adjacent rings. Typically, there are three links that connect adjacent rings and the links of one ring are circumferentially offset by about 60° from the links of an adjacent ring. While the links 31 typically are offset as indicated, this is not always the case. A central opening 40 in the proximal section 26 of the stent allows the passage of a catheter branch of the delivery system. Typically, and as will be described in more detail below, the expandable portion of the catheter will be a balloon similar to a dilatation-type balloon for conventional dilatation catheters. In the present invention, the stent 20 is configured such that the stent has a distal opening 36 and a proximal opening 38 that are in axial alignment and through which the balloon extends, and the central opening 40 which is adjacent the portal section 28 through which a balloon-less catheter branch extends. The stent is to be crimped tightly onto the expansion balloon and balloon-less catheter branch of such delivery system as will be described.

The rings 30 can be attached to each other by links 31 having various shapes, including straight links 32 or non-linear links 33 having curved portions. The non linear links can have undulating portions that are perpendicular (or offset) to the longitudinal axis of the stent and act as a hinge to enhance the flexibility of the stent. The links are not limited by any particular length or shape and can be a weld, laser fusion, or similar connection. Welds or laser fusion processes are particularly suited to stent patterns that are out of phase (the peaks point toward each other) as opposed to the in phase pattern (the peaks point in the same direction) shown in the drawings.

The rings 30 and links 31 can have variable thickness struts at various points in order to increase the radial strength of the stent, provide higher radiopacity so that the stent is more visible under fluoroscopy, and enhance flexibility in the portions where the stent has the thinnest struts. The stent also can have variable width struts, to vary flexibility, maximize vessel wall coverage at specific points, or to enhance the stent radiopacity. The variable thickness struts or variable width struts, which may be more radiopaque than other struts, can be positioned along the stent to help the physician position the stent during delivery and implantation in the bifurcated vessel.

The stent 20 can be formed in a conventional manner typically by laser cutting a tubular member or by laser cutting a pattern into a flat sheet, rolling it into a cylindrical body, and laser welding a longitudinal seam along the longitudinal edges of the stent. The stent can also be fabricated using conventional lithographic and etching techniques where a mask is applied to a tube or flat sheet. The mask is in the shape of the final stent pattern and is used for the purpose of protecting the tubing during a chemical etching process which removes material from unwanted areas. Electro discharge machining (EDM) can also be used for fabricating the stent, where a mold is made in the negative shape of the stent and is used to remove unwanted material by use of an electric discharge. The method of making stents using laser cutting processes or the other described processes are well known. The stent typically is made from a metal alloy and includes any of stainless steel, titanium, nickel-titanium (NiTi or nitinol of the shape memory or superelastic types), tantalum, cobalt-chromium, cobalt-chromium-vanadium, cobalt-chromium-tungsten, gold, silver, platinum, platinum-iridium or any combination of the foregoing metals and metal alloys. Any of the listed metals and metal alloys can be coated with a polymer containing fluorine-19 (19F) used as a marker which is visible under MRI. Portions of the stent, for example some of the links, can be formed of a polymer impregnated with 19F so that the stent is visible under MRI. Other compounds also are known in the art to be visible under MRI and also can be used in combination with the disclosed metal stent.

The stent also can be coated with a drug or therapeutic agent to assist in repair of the bifurcated vessel and may be useful, for example, in reducing the likelihood of the development of restenosis. Further, it is well known that the stent (usually made from a metal) may require a primer material coating to provide a substrate on which a drug or therapeutic agent is coated since some drugs and therapeutic agents do not readily adhere to a metallic surface. The drug or therapeutic agent can be combined with a coating or other medium used for controlled release rates of the drug or therapeutic agent. Examples of therapeutic agents that are available as stent coatings include rapamycin, actinomycin D (ActD), or derivatives and analogs thereof. ActD is manufactured by Sigma Aldrich, 1001 West Saint Paul Avenue, Milwaukee, Wisconsin 53233, or COSMEGEN, available from Merck. Synonyms of actinopmycin D include actinomycin, actinomycin IV, actinomycin 11, actinomycin X1, and actinomycin C1. Examples of agents include other antiproliferative substances as well as antineoplastic, antinflammatory, antiplatelet, anticoagulant, antifibrin, antithomobin, antimitotic, antibiotic, and antioxidant substances. Examples of antineoplastics include taxol (paclitaxel and docetaxel). Examples of antiplatelets, anticoagulants, antifibrins, and antithrombins include sodium heparin, low molecular weight heparin, hirudin, argatroban, forskolin, vapiprost, prostacyclin and prostacyclin analogs, dextran, D phe pro arg chloromethylketone (synthetic antithrombin), dipyridamole, glycoprotein, 11b/111a platelet membrane receptor antagonist, recombinant hirudin, thrombin inhibitor (available from Biogen), and 7E 3B® (an antiplatelet drug from Centocore). Examples of antimitotic agents include methotrexate, azathioprine, vincristine, vinblastine, fluorouracil, adriamycin, and mutamycin. Examples of cytostatic or antiproliferative agents include angiopeptin (a somatostatin analog from Ibsen), angiotensin converting enzyme inhibitors such as Captopril (available from Squibb), Cilazapril (available from Hoffman LaRoche), or Lisinopril (available from Merck); calcium channel blockers (such as Nifedipine), colchicine fibroblast growth factor (FGF) antagonists, fish oil (omega 3 fatty acid), histamine antagonist, Lovastatin (an inhibitor of HMG-CoA reductase, a cholesterol lowering drug from Merck), monoclonal antibodies (such as PDGF receptors), nitroprusside, phosphodiesterase inhibitors, prostaglandin inhibitor (available from Glazo), Seramin (a PDGF antagonist), serotonin blockers, steroids, thioprotease inhibitors, triazolopyrimidine (a PDGF antagonist), and nitric oxide. Other therapeutic substances or agents which may be appropriate include alpha-interferon, genetically engineered epithelial cells, and dexamethasone.

It should be understood that any reference in the specification or claims to a drug or therapeutic agent being coated on the stent is meant that one or more layers can be coated either directly on the stent or onto a primer material on the stent to which the drug or therapeutic agent readily attaches.

### The Stent Delivery Catheter Assembly

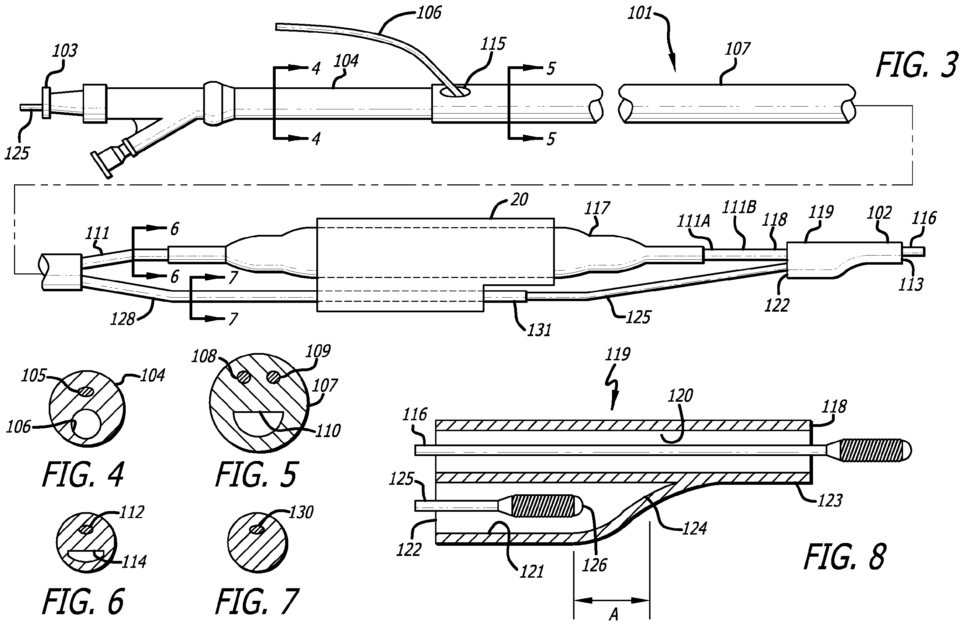

The stent is delivered by a catheter assembly. As shown in FIGS. 3-8, the stent 20 is mounted on catheter assembly 101 which has a distal end 102 and a proximal end 103. The catheter assembly includes a proximal shaft 104 which has a proximal shaft over-the-wire (OTW) guide wire lumen 105 and a proximal shaft inflation lumen 106 which extends therethrough. The proximal shaft OTW guide wire lumen is sized for slidably receiving an OTW guide wire. The inflation lumen extends from the catheter assembly proximal end where an indeflator or similar device is attached in order to inject inflation fluid to expand the balloon or expandable member as will be herein described. The catheter assembly also includes a mid shaft 107 having a mid-shaft OTW guide wire lumen 108 and a mid shaft rapid exchange (Rx) guide wire lumen 109. The proximal shaft OTW guide wire lumen 105 is in alignment with and an extension of the mid-shaft OTW guide wire lumen 108 for slidably receiving an OTW guide wire. The mid-shaft also includes a mid-shaft inflation lumen 110 which is in fluid communication with the proximal shaft inflation lumen 106 for the purpose of providing inflation fluid to the expandable balloons. There is an Rx proximal port or exit notch 115 positioned on the mid-shaft such that the Rx proximal port is substantially closer to the distal end 102 of the catheter assembly than to the proximal end 103 of the catheter assembly. While the location of the Rx proximal port may vary for a particular application, typically the port would be between 10 and 50 cm from the catheter assembly distal end 102. The Rx proximal port or exit notch provides an opening through which an Rx guide wire 116 exits the catheter and which provides the rapid exchange feature characteristic of such Rx catheters. The Rx port 115 enters the mid-shaft such that it is in communication with the mid shaft Rx guide wire lumen 109.

The catheter assembly 101 also includes a distal Rx shaft 111 that extends from the distal end of the mid shaft and which includes an Rx shaft Rx guide wire lumen 112, to the proximal end of the inner member 111A inside balloon 117. The distal Rx shaft 111 also contains an Rx shaft inflation lumen 114. The Rx shaft Rx guide wire lumen 112 is in alignment with the Rx guide wire lumen 109 for the purposes of slidably carrying the Rx guide wire 116. The Rx shaft inflation lumen 114 is in fluid communication with the mid shaft inflation lumen 110 for the purposes of carrying inflation fluid to the expandable member or balloon 117.

The catheter assembly also contains an Rx inner member 111A that extends from the distal end of the distal Rx shaft 111 to the Rx shaft distal port 113. The Rx inner member 111A contains an Rx guide wire lumen 111B. The Rx inner member guide wire lumen 111B is in alignment with the Rx shaft Rx guide wire lumen 112 for the purpose of slidably carrying the Rx guide wire 116. The Rx guide wire will extend through the Rx proximal port 115 and be carried through Rx guide wire lumen 109 and Rx shaft Rx guide wire lumen 112, and through Rx guide wire lumen 111B and exit the distal end of the catheter assembly at Rx shaft distal port 113.

The catheter assembly further includes a balloon 117 positioned adjacent the distal end of the catheter assembly and a distal tip 118 at the distal end of the Rx shaft. Further, a coupler 119 is associated with distal Rx shaft 111 such that the Rx shaft Rx guide wire lumen 112 extends through the coupler, with the distal port 113 being positioned at the distal end of the coupler. The coupler has an Rx guide wire lumen 120 that is an extension of and in alignment with Rx lumen 111B. The coupler 119 further includes a blind lumen 121 for receiving and carrying an OTW guide wire (or joining mandrel) 125. The blind lumen includes a blind lumen port 122 for receiving the distal end of the OTW guide wire (or joining mandrel) 125 and a dead end lumen 124 positioned at the coupler distal end 123. The coupler blind lumen 121 will carry the distal end of a guide wire (either the distal end of the OTW guide wire (or joining mandrel) 125 or an Rx guide wire (or joining mandrel) 116 as will be further described herein) during delivery of the catheter assembly through the vascular system and to the area of a bifurcation. The blind lumen is approximately 3 to 20 mm long, however, the blind lumen can vary in length and diameter to achieve a particular application or to accommodate different sized guide wires having different diameters. Since the coupler moves axially relative to the shaft it is not connected to, the guide wire that resides in the blind lumen 121 of the coupler slides axially relative to the coupler during delivery of the catheter assembly through the vascular system and tortuous anatomy so that, additional flexibility is imported to the tips making it easier to track through tortuous circuitry. A distance "A" should be maintained between the distal end 126 of the OTW guide wire 125 and the dead end 124 of the blind lumen. The distance "A" can range from approximately 0.5 to 5.0 mm, however, this range may vary to suit a particular application. Preferably, distance "A" should be about 0.5 mm to about 2.0 mm.

The catheter assembly 101 also includes an OTW shaft 128 which branches out from the distal end of mid-shaft 107. The OTW shaft is shorter than the balloon 117 and is positioned substantially adjacent to the long balloon. The OTW shaft 128 also includes an OTW lumen 130 that is in alignment with the mid-shaft OTW guide wire lumen 108 and proximal shaft OTW guide wire lumen 105. Thus, an OTW lumen extends from one end of the catheter assembly to the other and extends through the OTW shaft 128. An OTW shaft distal port 131 is at the distal end of the OTW lumen 130. In this particular embodiment, an OTW guide wire 125 would extend from the proximal end 103 of the catheter assembly and through proximal shaft OTW guide wire lumen 105, mid shaft OTW guide wire lumen 108, OTW lumen 130 and out distal port 131 where it would extend into the coupler 119, and more specifically into blind lumen 121 through blind lumen port 122.

In order for the catheter assembly 101 to smoothly track and advance through tortuous vessels, it is preferred that the OTW lumen 130 be substantially aligned with the blind lumen 121 of coupler 119. In other words, as the OTW guide wire extends out of the OTW lumen 130, it should be aligned without bending more than about ±10° so that it extends fairly straight into the coupler blind lumen 121. If the OTW lumen 120 and the coupler blind lumen 121 are not substantially aligned, the pushability and the trackability of the distal end of the catheter assembly may be compromised and the physician may feel resistance as the catheter assembly is advanced through tortuous vessels, such as the coronary arteries.

In an alternative embodiment, as will be explained more fully herein, a mandrel (stainless steel or nickel titanium wire is preferred) resides in the OTW guide wire lumens 105,108,130, and extends into blind lumen 121. The mandrel is used in place of an OTW guide wire until the catheter assembly has been positioned near the bifurcated vessel, at which time the mandrel can be withdrawn from the vascular system and the OTW guide wire advanced through the OTW guide wire lumens to gain access to the side branch vessel. This will be described more fully in the section related to delivering and implanting the stent.

The catheter assembly 101 can be dimensioned for various applications in a patient's vascular system. Such dimensions typically are well known in the art and can vary, for example, for various vessels being treated such as the coronary arteries, peripheral arteries, the carotid arteries, and the like. By way of example, the overall length of the catheter assembly for treating the coronary arteries typically is approximately 135 to 150 cm. Further, for stent delivery in the coronary arteries at a bifurcated vessel, the working surface or the stent carrying surface of the balloon 117 can be about 18.5 mm for use with an 18 mm long stent. The length of the balloon-less shaft 128 will depend upon the type of trap door stent 20 that is being implanted. The lengths of the various shafts, including proximal shaft 104, mid shaft 107, distal Rx shaft 111, and OTW shaft 128 are a matter of choice and can be varied to suit a particular application.

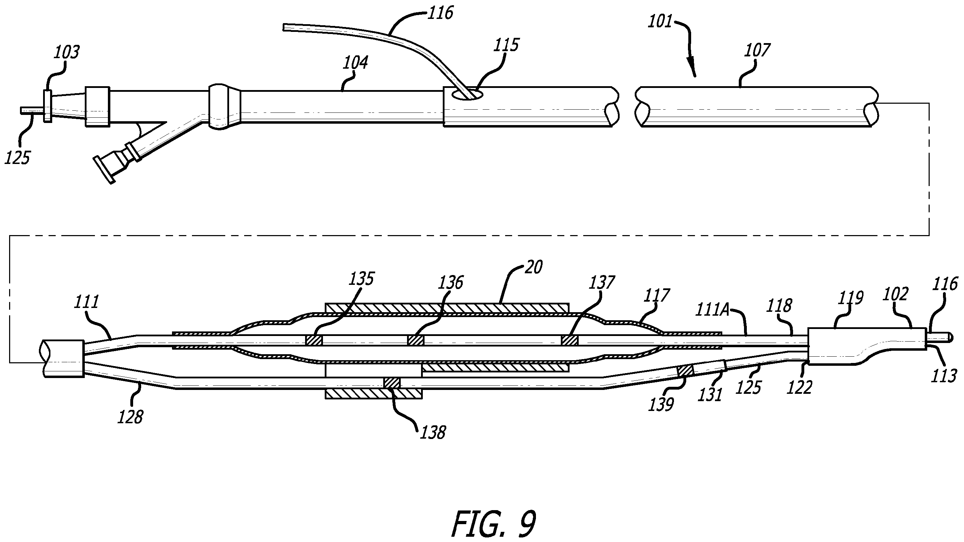

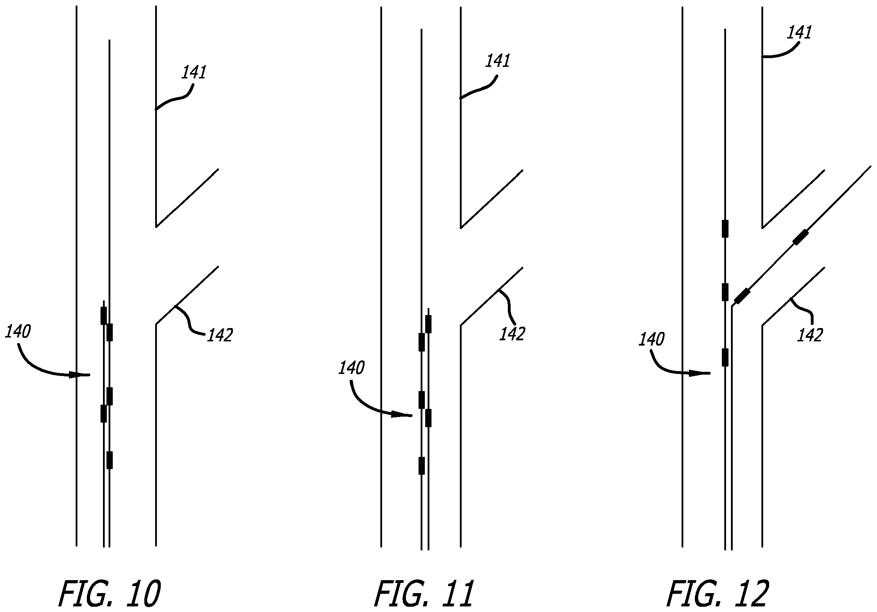

As shown in FIG. 9, radiopaque markers 135-139 are placed on the catheter assembly to help the physician identify the location of the distal end of the catheter in relation to the target area for stent implantation. The radiopaque markers are positioned along the balloon 117 at (135) the proximal end of the stent, adjacent (136) the side branch portal and at (137) the distal end of the stent and along the balloon-less branch 128, immediately proximal (138) to the side branch portal and at (139) its distal end. Such placement, and more particularly, the relative placement of the five markers, readily allows the longitudinal and rotational orientation of the catheter, and hence stent, to be discerned under fluomgraphy. FIG. 10 illustrates the stent and catheter assembly 140 within a main branch vessel 141 in an "out-of-phase" orientation relative to the side branch vessel 142 wherein the balloon-less branch and hence the side branch portal of the stent is rotated 180° relative to the side branch vessel. FIG. 11 illustrates the stent and catheter assembly "in-phase" with the side branch vessel while FIG. 12 illustrates the two branches properly extended into the two vessel branches.

A radiopaque marker for use on a variety of devices is preferably flexible, highly radiopaque and is easily attachable to such devices by melt bonding. These properties allow markers to be of minimal thickness and thereby minimize the effect the marker has on the overall profile and stiffness of the device to which it is to be attached.

In order to achieve the high fill ratios that are necessary to attain the desired radiopacity and in order to do so without compromising the compoundability and workability of the polymeric material nor its ultimate strength and flexibility, a number of different parameters have been found to be of importance. More specifically, both the particle shape and particle size of the radiopaque agent must be carefully controlled while the inclusion of a wetting agent such as MA-g-PO in the polymer blend is critical. An antioxidant may additionally be included in an effort to reduce the adverse effect the high processing temperatures and shear stresses may have on polymer properties.

A number of polymeric materials are well suited for use in the manufacture of the radiopaque markers. The material preferably comprises a low durometer polymer in order to render the marker sufficiently flexible so as not to impair the flexibility of the underlying medical device component to which the finished marker is to be attached. Additionally, the polymer is preferably compatible with the polymeric material of which the component is constructed so as to allow the marker to be melt bonded in place. For example, in one embodiment, the polymeric marker and at least an outer layer of the catheter shaft are formed of the same class of the polymers (e.g., polyamides) so that they are melt bondable together. In another embodiment, the polymeric markers are installed on a dissimilar class of polymeric substrate, and are retained in position by adhesion or dimensional interference. The polymer must also impart sufficient strength and ductility to the marker compound so as to facilitate its extrusion and forming into a marker, its subsequent handling and attachment to a medical device and preservation of the marker's integrity as the medical device is flexed and manipulated during use. Examples of such polymers include but are not limited to polyamide copolymers like Pebax, polyetherurethanes like Pellethane, polyester copolymers like Hytrel, olefin derived copolymers, natural and synthetic rubbers like silicone and Santoprene, thermoplastic elastomers like Kraton and specialty polymers like EVA and ionomers, etc. as well as alloys thereof. A Shore durometer of not greater than about 63D to about 25D is preferred. The preferred polymer for use in the manufacture of a marker is polyether block polyamide copolymer (PEBAX), with a Shore durometer of about 40D. However, other classes of polymers allowing for lower durometers may be used in the radiopaque markers, such as polyurethanes, which may provide greater flexibility.

A number of different metals are well known to be radiographically dense and can be used in a pure or alloyed form to mark medical devices so as to render them visible under fluoroscopic inspection. Commonly used metals include but are not limited to platinum, gold, iridium, palladium, rhenium and rhodium. Less expensive radiopaque agents include tungsten, tantalum, silver and tin, of which tungsten is most preferred.

The control of particle size has been found to be of critical importance for achieving the desired ultra high fill ratios. While efforts to increase fill ratios have previously utilized small average particle sizes (1 micron or less) so as to minimize the ratio of particle size to as-extruded wall thickness, it has been found that higher fill percentages can be realized with the use of somewhat larger average particles sizes. It is desirable in the formulation of high fill ratio compounds to have the following attribute: 1) uniform distribution of the filler particles, and 2) continuity of the surrounding polymer matrix, and 3) sufficient spacing between filler particles so that the polymer matrix provides ductility to the bulk mixture to impart processability in both the solid and molten state.

The use of larger average particle sizes results in greater spacing between filler particles at a given percentage, thus maintaining processability during compounding and especially subsequent extrusion coating. The upper limit of average particle size is determined by the wall thickness of the coating and the degree of non-uniformity tolerable (i.e., surface defects). It has been found that a particle size distribution having an average particle size range of at least 2 microns to 10 microns and a maximum particle size of about 20 microns yields the desired fill ratio and provides for a smooth surface in the marker made wherefrom.

The control of particle shape has also been found to be of critical importance for achieving the desired ultra high fill ratios. Discrete particles of equiaxed shape have been found to be especially effective, as individual particles of irregular shape, including agglomerations of multiple particles, have been found to adversely impact the surface, and thus, the maximum fill ratio that is attainable.

It has also been found that the process by which certain metal powders are produced has a profound effect on the shape of the individual particles. In the case of metallic tungsten, the powders may be formed by the reduction of powdered oxides through either "rotary," "pusher" or "atomization" processing. Of these processes, "rotary" processing has been found to yield the least desirable shape and size distribution as partial sintering causes coarse agglomerates to be formed which do not break up during compounding or extrusion and thus adversely effect the marker manufactured therefrom. Atomized powders have been reprocessed by melting and resolidifying "rotary" or "pusher" processed powders and result in generally equiaxed, discrete particles which are suitable for use in the present invention. "Pusher" processed powders are preferred due to their low cost and discrete, uniformly shaped particles.

In order for the polymer to most effectively encapsulate individual radiopaque particles, it is necessary for a low-energy interface to exist between such particles and the polymer so as to enable the polymer to "wet" the surface of the particles. The materials should have similar surface energies to be compatible. For materials which do not naturally have similar surface energies, compatibility can be promoted by generating a similar surface energy interface, i.e., a surface energy interface which is intermediate between the natural surface energies of the materials. Certain additives such as surfactants and coupling agents may serve as wetting agents and adhesion promoters for polymer/metal combinations that are not naturally compatible. It has been found that additives containing maleic anhydride grafted to a polyolefin backbone provide a significant benefit in this regard wherein materials commercially available as Lotader 8200 (having LLDPE Backbone) and Licomont AR504 (having PP backbone) were found to be particularly effective for use with tungsten/Pebax combinations. Emerging extrusions were found to be less susceptible to breakage, and the melt viscosity during compounding was lower as was manifested by a reduction in torque exerted during the extrusion process. The use of such additives allowed compounds with higher fill ratios to be successfully produced.

The inclusion of an antioxidant in the marker composition has also been found to be of benefit. Commercially available antioxidants such as Irganox B225 or Irganox 1010, have been found to minimize degradation (i.e., reduction in molecular weight) of the polymer matrix as it is exposed to the multiply heat and shear histories associated with the compounding, extrusion, and bonding processes.

The compound used for the manufacture of the marker is preferably made by first blending the polymer resin and wetting agent, and optionally, an antioxidant such as by tumble mixing after which such blend is introduced into a twin-screw extruder via a primary feeder. The feed rate is carefully controlled in terms of mass flow rate to ensure that a precise fill ratio is achieved upon subsequent combination with the radiopaque agent. The heat that the materials are subjected as they are conveyed through the extruder causes the polymer to melt to thereby facilitate thorough homogenization of all of the ingredients. The radiopaque agent powder, selected for its uniform particle shape and controlled particle size distribution as described above is subsequently introduced into the melt stream via a secondary feeder, again at a carefully controlled mass flow rate so as to achieve the target fill ratio. The solid powder, molten polymer and additives are homogenized as they are conveyed downstream and discharged through a die as molten strands which are cooled in water and subsequently pelletized. The preferred extrusion equipment employs two independent feeders as introduction of all components through a single primary feeder would require significantly higher machine torques and result in excessive screw and barrel wear. The powder feeder is preferentially operated in tandem with a sidefeeder device, which in turn conveys the powder through a sealed main barrel port directly into the melt stream. A preferred composition comprises a fill ratio of at least 90.8 weight percent of tungsten (H.C. Starck's Kulite HC600s, HC180s and KMP-103JP) to Pebax 40D. A maleic anhydride source in the form of Licomont AR504 is initially added to the polymer resin at the rate of approximately 3 pphr while an antioxidant in the form of Ciba Geigy Irganox B225 at the rate of approximately 2 pphr (parts per hundred relative to the resin). The temperature to which materials are subjected to in the extruder is about 221°C.

Once the marker material has been compounded, the marker can be fabricated in suitable dimensions by an extrusion coating process. While free extrusion is possible, this method is problematic due to the high fill ratios of the polymeric materials. Extrusion onto a continuous length of beading has been found to lend the necessary support for the molten extrudate to prevent breakage. The support beading may take the form of a disposable, round mandrel made of PTFE (Teflon) coated stainless steel wire or other heat resistant material that does not readily bond to the extrudate. By additionally limiting the area draw down ratio (ADDR) to below 10:1 the tungsten-laden melt can successfully be drawn to size by an extrusion puller. The beading provides the added benefit of fixing the inner diameter and improving overall dimensional stability of the final tungsten/polymer coating. Extrusions of a 91.3 weight percent fill ratio tungsten/Pebax composition described above over 0.546mm (0.0215") diameter PTFE beading were successfully drawn down to a wall thickness of 0.064mm (0.0025") to yield a marker properly sized for attachment to for example a 0.56mm (0.022") diameter inner member of balloon catheter. Also, extrusion coatings of 91% compound over 0.18mm (.007") teflon coated stainless steel wire were successfully drawn down to single wall thicknesses of 0.05mm (.002") to make guidewire coatings.

In one arrangement once the extrudate has cooled, the extrusion is simply cut to the desired lengths (e.g., 1 to 1.5 mm) of the individual markers, such as with the use of a razor blade and reticle, preferably with the beading still in place to provide support during cutting. The beading remnant is subsequently ejected and the marker is slipped onto a medical device or a particular component thereof. In one arrangement, a segment of the extrudate is hot die necked with the beading inside to resize the outer diameter and wall thickness of the extrudate prior to cutting into individual markers. For example, an extrudate, having an inner diameter of about 0.546 ± 0.013mm (about 0.0215 ± 0.0005 inch) and an outer diameter of about 0.699 ± 0.025mm (about 0.0275 ± 0.001 inch), is hot die necked to an outer diameter of about 0.673mm (about 0.0265 inch) to produce a double wall thickness of about 0.127 ± 0.013mm (about 0.005 ± 0.0005 inch). To minimize part to part variability in double wall thickness, the actual hot die size may be selected based upon the actual beading diameter prior to hot die necking.

Finally, the marker is attached to the underlying substrate, preferably with the use of heat shrink tubing and a heat source (hot air, laser, etc.) wherein the heat (∼171-210°C) simultaneously causes the marker to melt and the heat shrink tubing to exert a compressive force on the underlying molten material. To prevent extensive dimensional changes (e.g., thinning) of the polymeric marker, the temperatures used are below the melting temperature, thereby relying on heat and pressure to soften the marker and generate an adhesive bond with the underlying substrate. For markers formed of PEBAX 40D, the temperature used is about 120-135°C. Heat bonding a marker onto an underlying component provides the added benefit of slightly tapering the edges of the marker to reduce the likelihood of catching an edge and either damaging the marker or the medical device during assembly or handling of the medical device.

A marker formed as per the above described compounding, fabricating and assembling processes, having a fill ratio of 91.3 weight percent (36.4 volume percent) with a wall thickness of 0.064mm (0.0025") has been shown to have dramatically more radiopacity than commercially available 80 weight percent compounds and comparable to the radiopacity of 0.0318mm (0.00125 inch) thick conventional Platinum/10% Iridium markers. The radiopacity is a function of the total volume of radiopaque material present in the marker (i.e., the product of the volume % and the volume of the marker). In a presently preferred embodiment, the marker is about 1.5mm long and has a double wall thickness of about 0.114 to about 0.140mm (about 0.0045 to about 0.0055 inch) and a fill ratio of about 90.8 to about 93.2 weight percent of tungsten, which provides a volume of radiopaque material substantially equal to the volume of Platinum/10% Iridium in a 1.0mm long 0.064mm, (0.0025 inch) thick (double wall) conventional Platinum/Iridium marker band. Preferably, the volume of radiopaque material is not less than about 30%, and the double wall thickness of the marker is at least about 0.10mm (0.004 inch), to provide sufficient radiopacity. However, as discussed above, the ability to increase the volume of the marker by increasing the wall thickness of the marker is limited by the resulting increase in profile and stiffness. In a presently preferred embodiment, the double wall thickness of the marker is not greater than about 0.15mm (0.006 inch).

The materials used to construct the catheter assembly are known in the art and can include for example various compositions of PEBAX, PEEK (polyetherketone), urethanes, PET or nylon for the balloon materials (polyethylene terephathalate) and the like. Other materials that may be used for the various shaft constructions include fluorinated ethylenepropylene resins (FEP), polytetrafluoroethylene (PTFE), fluoropolymers (Teflon), Hytrel polyesters, aromatic polymers, block co polymers, particularly polyamide/polyesters block co-polymers with a tensile strength of at least 41.37 MPa (6,000 psi) and an elongation of at least 300%, and polyamide or nylon materials, such as Nylon 12, with a tensile strength of at least 103.42 MPa (15,000 psi). The various shafts are connected to each other using well known adhesives such as

Loctite or using heat-shrink tubing over the joint of two shafts, of which both methods are well known in the art. Further, any of the foregoing catheter materials can be combined with a compound that is visible under MRI, such as 19F, as previously discussed herein.

### Delivering and Implanting the Stent

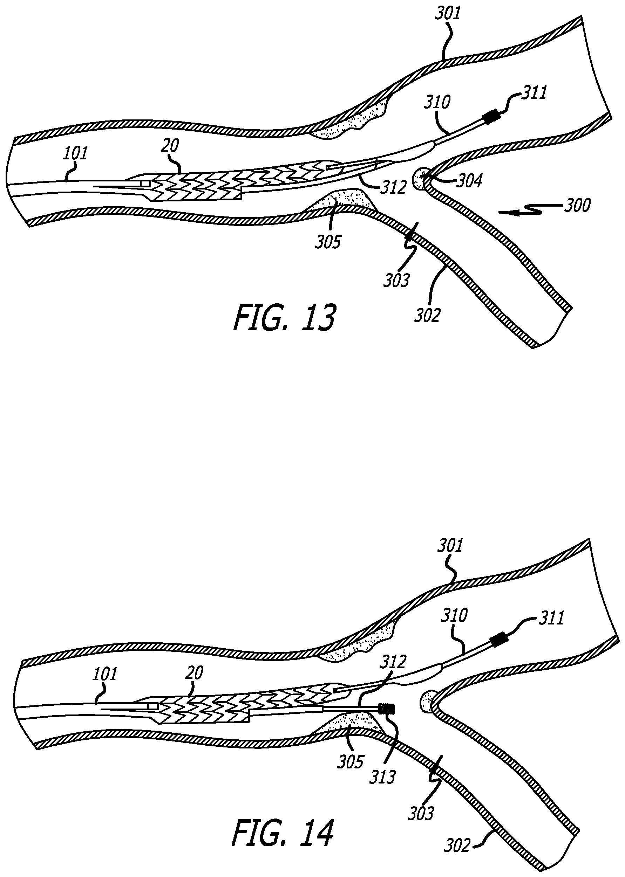

Referring to FIGS. 13 - 17, the bifurcated catheter assembly provides a balloon carrying branch and a balloon-less branch in parallel which can be advanced into separate passageways of an arterial bifurcation where upon inflation of the balloon causes the stent to expand and a side branch portal to be opened. As shown in the drawings, bifurcation 300 typically includes a main vessel 301 and a side branch vessel 302 with the junction between the two referred to as the carina 304. Typically, plaque 305 will develop in the area around the junction of the main vessel and the side branch vessel and, as previously described with the prior art devices, is difficult to stent without causing other problems such as portions of the stent extending into the blood flow path jailing a portion of the side branch vessel, or causing plaque to shift at the carina and subsequently occlude the vessel.

During delivery the catheter assembly 101 is advanced through a guiding catheter (not shown) in a known manner. Once the distal end 102 of the catheter reaches the ostium to the coronary arteries, the Rx guide wire 310 is advanced distally into the coronary arteries (or any other bifurcated vessel) so that the Rx guide wire distal end 311 extends past the opening to the side branch vessel 303. (In most cases, the main vessel will have been predilated in a known manner prior to delivery of the trap door stent. In these cases, the Rx guide wire will have been left in place across and distal to the target site prior to loading the catheter assembly onto the Rx guide wire for advancement to the target site.) After the distal end of the Rx guide wire is advanced into the main vessel past the opening to the side branch vessel, the catheter is advanced over the Rx guide wire so that the catheter distal end 102 is just proximal to the opening to the side branch vessel. Up to this point in time, the OTW guide wire 312 (or mandrel) remains within the catheter and within coupler 119 keeping the tips and catheter branches joined. More specifically, the OTW guide wire remains within the OTW guide wire lumens 105,108, and 130 as previously described. The distal end of the OTW guide wire 313 is positioned within coupler blind lumen 121 during delivery and up to this point in time. As the catheter is advanced through tortuous coronary arteries, the OTW guide wire distal end 313 should be able to slide axially a slight amount relative the coupler blind lumen to compensate for the bending of the distal end of the catheter. As the catheter distal end moves through tight twists and turns, the coupler moves axially relative to the balloon shaft that it is not attached to thereby creating relative axial movement with the OTW guide wire. Stated differently, the coupler moves axially a slight amount while the OTW guide wire remains axially fixed (until uncoupled) relative to the catheter shaft. If the OTW guide wire were fixed with respect to the coupler at the distal end, it would make the distal end of the catheter stiffer and more difficult to advance through the coronary arteries, and may cause the distal end of the catheter to kink or to be difficult to push through tight turns. Thus, the coupler moves axially relative to the distal end of the OTW guide wire in a range of approximately 0.5 mm up to about 5.0 mm. Preferably, the coupler moves axially relative to the OTW guide wire distal end 313 about 0.5 mm to about 2.0 mm. The amount of axial movement will vary depending on a particular application and the severity of the tortuousity. The proximal end of the OTW guide wire (or joining wire or mandrel) should be removably fixed relative to the catheter shaft during delivery so that the distal end of the OTW guide wire does not prematurely pull out of the coupler. The distal end of the OTW guide wire still moves axially a small amount within the coupler as the distal end of the catheter bends and twists in negotiating tortuous anatomy.

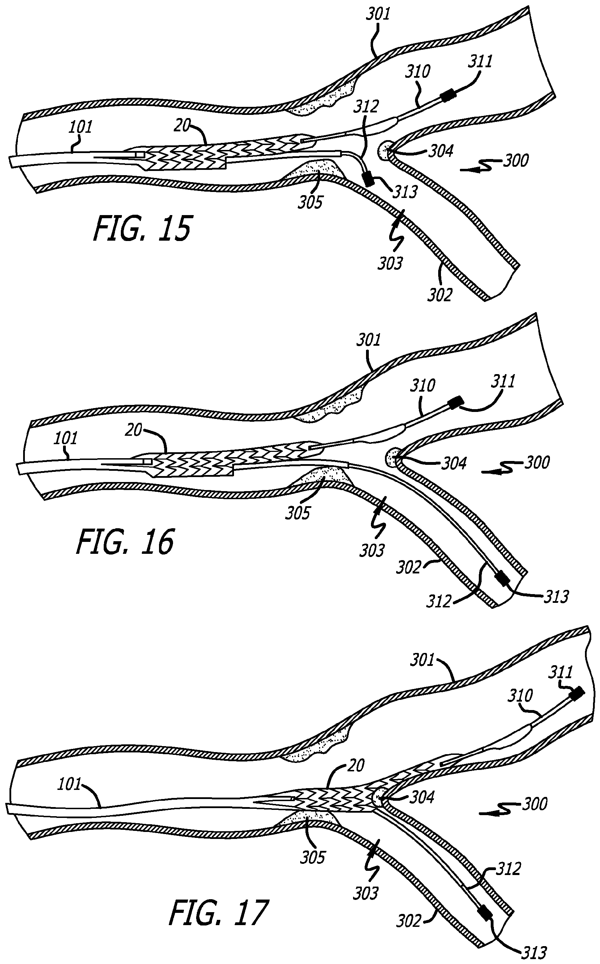

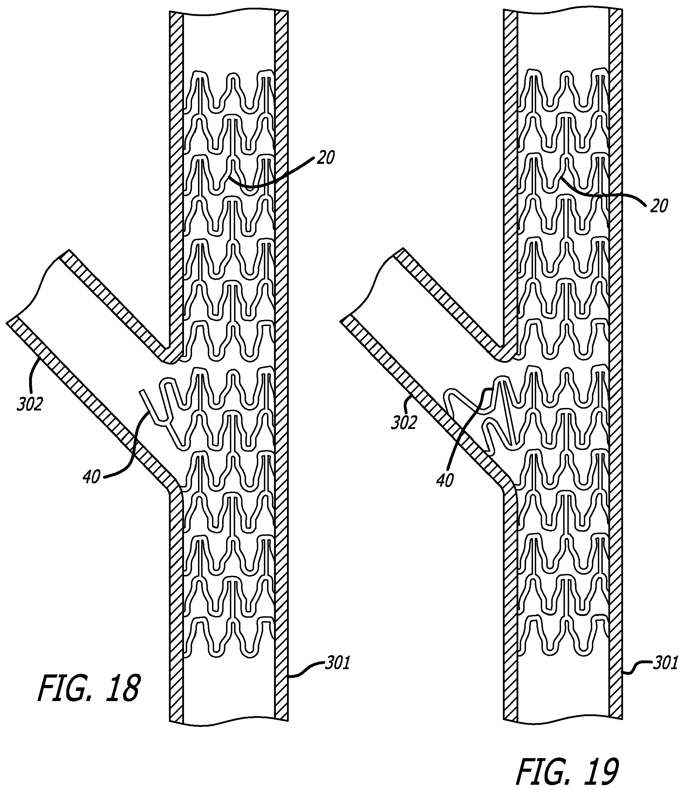

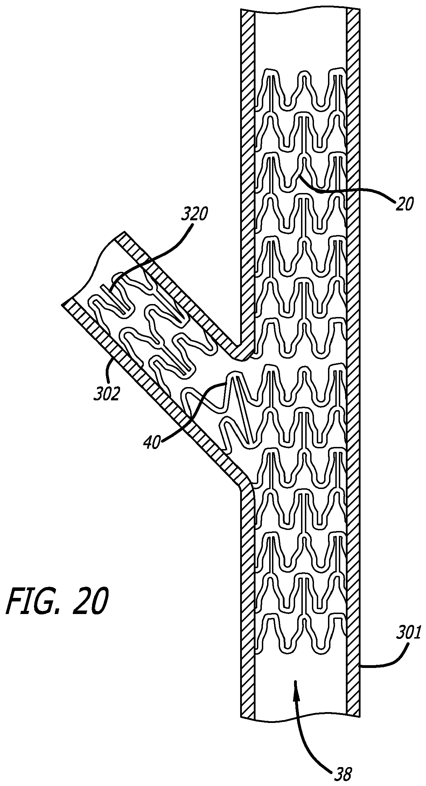

As previously disclosed, when the radiopaque markers 135-139 are arranged as shown in FIG. 11, the OTW guide wire can be released from the coupler. As shown for example in FIG. 14, the OTW guide wire 312 next is withdrawn proximally so that the OTW guide wire distal end 313 is removed from the coupler blind lumen 121. As shown in FIG. 15, the OTW guide wire next is advanced distally into the side branch vessel 302, extending past the opening to the side branch vessel 303 and advancing distally into the vessel for a distance as shown in FIG. 16. Once the Rx guide wire 310 is in position in the main vessel, and the OTW guide wire 312 is in position in the side branch vessel, this will have a tendency to impart a slight separation between the balloon 117 and the balloon-less branch 128. As shown in FIG. 17, the catheter assembly 101 is advanced distally over the Rx guide wire and the OTW guide wire and, as the assembly is further advanced, the balloon 117 continues to separate from the balloon-less branch 128 as each advances into the main vessel 301 and the side branch vessel 302 respectively. As the assembly continues to advance distally, it will reach the point where side branch portal 40 on the stent 20 is adjacent the opening to the side branch vessel 303. At this point, the catheter assembly can no longer be advanced distally since the stent is now pushing up against the opening to the side branch vessel. The balloon 117 is next inflated simultaneously to expand the stent 20 into the main vessel and urge the balloon-less side branch radially into the opening to the side branch vessel. As shown in FIG. 18, a portion of the central section 28 of the stent will expand into the side branch vessel to create a path for subsequent introduction of a kissing balloon with which the central opening 40 of the stent can be opened fully to clear a blood flow path through the proximal opening of the stent 38 and through the central opening 40 into the side branch vessel as is shown in FIG. 19. As shown in FIG. 20, a second stent 320 can be implanted in the side branch vessel 302 such that it abuts central opening 40 of stent 20.

While particular forms of the invention have been illustrated and described, it will be apparent to those skilled in the art that various modifications can be made without departing from the scope of the invention. Accordingly, it is not intended that the invention be limited except by the appended claims.

## Claims

1. A stent and catheter assembly for treating a bifurcation, comprising:
a catheter (101) having a first shaft (111) and a second shaft (128), wherein said shafts (111,128) are arranged parallel to one another and, wherein said first shaft (111) includes an inflatable balloon (117) and has three radiopaque markers (135,136,137) affixed thereto and said second shaft (128) is devoid of a balloon and has two radiopaque markers (138,139) affixed thereto, said first shaft (111) having a first guide wire lumen (112) extending therethrough and a coupler (119) for receiving a guide wire disposed near its distal end and said second shaft (128) having a second guide wire lumen (130) extending therethrough; and
a stent (20) having a proximal end (22), a distal end (23) and a side branch portal (40) positioned therebetween, wherein said stent (20) is positioned about said catheter (101) such that the catheter's first shaft (111) extends out through said stent's distal end (23) and the catheter's second shaft (128) extends out through said branch portal (40),
the radiopaque markers (135,136,137) affixed to the first shaft (111) being positioned along the balloon (117) at the proximal end of the stent (22), adjacent the side branch portal (40) and at the distal end of the stent (23) **characterised in that** the radiopaque markers (138, 139) affixed to the second shaft (128) being positioned immediately proximal to the side branch portal (40) and at said second shaft's distal end (13) thereby allowing the longitudinal and rotational orientation of the catheter (101) and the stent (20) to be discerned under fluoroscopy.

2. The stent and catheter assembly of claim 1, wherein said first guide wire lumen (112) has a first guide wire (116) extending therethrough and said second guide wire lumen (130) has a second guide wire (125) extending therethrough with a distal tip (126) slidably received in said coupler (119).

3. The stent and catheter assembly of claim 1, wherein one of said guide wire lumens (112,130) is an over-the-wire (OTW) guide wire lumen.

4. The stent and catheter assembly of claim 1, wherein one of said guide wire lumens (112,130) is a rapid-exchange (Rx) guide wire lumen.

5. The stent and catheter assembly of claim 1, wherein one of said guide wire lumens (130) is an over-the-wire (OTW) guide wire lumen and the other of said guide wire lumens (112) is a rapid-exchange (Rx) guide wire lumen.

6. The stent and catheter assembly of claim 5, wherein said first guide wire lumen (112) is an rapid-exchange (Rx) guide wire lumen.

## Patentansprüche

1. Stent-Katheter-System zur Behandlung einer Verzweigung, das aufweist:
einen Katheter (101), der einen ersten Schaft (111) und einen zweiten Schaft (128) aufweist, wobei die Schäfte (111, 128) parallel zueinander angeordnet sind und wobei der erste Schaft (111) einen aufblasbaren Ballon (117) und drei daran befestigte röntgenstrahlundurchlässige Markierungen (135, 136, 137) aufweist und der zweite Schaft (128) keinen Ballon und zwei daran befestigte röntgenstrahlundurchlässige Markierungen (138, 139) aufweist, der erste Schaft (111) ein sich durch ihn erstreckendes erstes Führungsdrahtlumen (112) und ein nahe seinem distalen Ende angeordnetes Verbindungsstück (119) zum Aufnehmen eines Führungsdrahtes aufweist und der zweite Schaft (128) ein sich durch ihn erstreckendes zweites Führungsdrahtlumen (130) aufweist; und
einen Stent (20), der ein proximales Ende (22), ein distales Ende (23) und ein dazwischen angeordnetes Seitenastportal (40) aufweist, wobei der Stent (20) so um den Katheter (101) angeordnet ist, dass sich der erste Schaft (111) des Katheters durch das distale Ende (23) des Stents hindurch erstreckt und sich der zweite Schaft (128) des Katheters durch das Seitenastportal (40) hindurch erstreckt,
wobei die an dem ersten Schaft (111) angebrachten röntgenstrahlundurchlässigen Markierungen (135, 136, 137) entlang des Ballons (117) am proximalen Ende (22) des Stents, angrenzend an das Seitenastportal (40) und am distalen Ende (23) des Stents angeordnet sind,
**dadurch gekennzeichnet, dass** die an dem zweiten Schaft (128) angebrachten röntgenstrahlundurchlässigen Markierungen (138, 139) unmittelbar proximal zu dem Seitenastportal (40) und an dem distalen Ende (13) des zweiten Schafts angeordnet sind, wodurch die Längsorientierung und Rotationsausrichtung des Katheters (101) und des Stents (20) durch Fluoroskopie erkannt werden können.

2. Stent-Katheter-Anordnung nach Anspruch 1, wobei das erste Führungsdrahtlumen (112) einen sich durch dieses erstreckenden ersten Führungsdraht (116) aufweist und das zweite Führungsdrahtlumen (130) einen sich durch dieses erstreckenden zweiten Führungsdraht (125) aufweist, wobei eine distale Spitze (126) in dem Verbindungsstück (119) verschiebbar aufgenommen ist.

3. Stent-Katheter-Anordnung nach Anspruch 1, wobei eines der Führungsdrahtlumen (112, 130) ein Over-the-wire (OTW)-Führungsdrahtlumen ist.

4. Stent-Katheter-Anordnung nach Anspruch 1, wobei eines der Führungsdrahtlumen (112, 130) ein Rapid-Exchange (Rx)-Führungsdrahtlumen ist.

5. Stent-Katheter-Anordnung nach Anspruch 1, wobei eines der Führungsdrahtlumen (130) ein Over-the-wire (OTW)-Führungsdrahtlumen ist und das andere Führungsdrahtlumen (112) ein Rapid-Exchange (Rx)-Führungsdrahtlumen ist.

6. Stent-Katheter-Anordnung nach Anspruch 5, wobei das erste Führungsdrahtlumen (112) ein Rapid-Exchange (Rx)-Führungsdrahtlumen ist.

## Revendications

1. Ensemble cathéter et endoprothèse vasculaire pour traiter une bifurcation, comprenant :
un cathéter (101) ayant une première tige (111) et une deuxième tige (128), où lesdites tiges (111, 128) sont agencées parallèlement l'une à l'autre et, où ladite première tige (111) comporte un ballonnet gonflable (117) et présente trois marqueurs radio-opaques (135, 136, 137) fixés à celle-ci et ladite deuxième tige (128) est dépourvue d'un ballonnet et présente deux marqueurs radio-opaques (138, 139) fixés à celle-ci, ladite première tige (111) ayant une première lumière de fil-guide (112) s'étendant à travers celle-ci et un coupleur (119) pour recevoir un fil-guide disposé à proximité de son extrémité distale et ladite deuxième tige (128) ayant une deuxième lumière de fil-guide (130) s'étendant à travers celle-ci ; et
une endoprothèse vasculaire (20) ayant une extrémité proximale (22), une extrémité distale (23) et une porte de ramification latérale (40) positionnée entre elles, où ladite endoprothèse vasculaire (20) est positionnée autour dudit cathéter (101) de sorte que la première tige du cathéter (111) s'étende vers l'extérieur à travers ladite extrémité distale de l'endoprothèse vasculaire (23) et la deuxième tige du cathéter (128) s'étende vers l'extérieur à travers ladite porte de ramification (40),
les marqueurs radio-opaques (135, 136, 137) fixés à la première tige (111) étant positionnés le long du ballonnet (117) au niveau de l'extrémité proximale de l'endoprothèse vasculaire (22), de manière adjacente à la porte de ramification latérale (40) et au niveau de l'extrémité distale de l'endoprothèse vasculaire (23), **caractérisé en ce que** les marqueurs radio-opaques (138, 139) fixés à la deuxième tige (128) étant positionnés à proximité immédiate de la porte de ramification latérale (40) et au niveau de l'extrémité distale de ladite deuxième tige (13), permettant ainsi à l'orientation longitudinale et rotationnelle du cathéter (101) et de l'endoprothèse vasculaire (20) d'être discernée sous radioscopie.

2. Ensemble cathéter et endoprothèse vasculaire de la revendication 1, dans lequel ladite première lumière de fil-guide (112) présente un premier fil-guide (116) s'étendant à travers celle-ci et ladite deuxième lumière de fil-guide (130) présente un deuxième fil-guide (125) s'étendant à travers celle-ci avec une pointe distale (126) reçue en coulissement dans ledit coupleur (119).

3. Ensemble cathéter et endoprothèse vasculaire de la revendication 1, dans lequel l'une desdites lumières de fil-guide (112, 130) est une lumière de fil-guide à implantation sur fil (OTW).

4. Ensemble cathéter et endoprothèse vasculaire de la revendication 1, dans lequel l'une desdites lumières de fil-guide (112, 130) est une lumière de fil-guide à échange rapide (Rx).

5. Ensemble cathéter et endoprothèse vasculaire de la revendication 1, dans lequel l'une desdites lumières de fil-guide (130) est une lumière de fil-guide à implantation sur fil (OTW) et l'autre desdites lumières de fil-guide (112) est une lumière de fil-guide à échange rapide (Rx).

6. Ensemble cathéter et endoprothèse vasculaire de la revendication 5, dans lequel ladite première lumière de fil-guide (112) est une lumière de fil-guide à échange rapide (Rx).
